# EUROPÄISCHE PATENTSCHRIFT

(11) **EP 0 381 785 B1**
(45) Veröffentlichungstag und Bekanntmachung des Hinweises auf die Patenterteilung: **18.11.1993**
(21) Anmeldenummer: 89102031.5
(22) Anmeldetag: 06.02.1989
(51) Int. Cl.: A61B 6/00

(54) **Rotierende Datenübertragungsvorrichtung**
Rotating data transmission device
Dispositif tournant pour la transmission de données

(43) Veröffentlichungstag der Anmeldung: 16.08.1990
(73) Patentinhaber: SIEMENS AKTIENGESELLSCHAFT, 80333 München (DE)
(72) Erfinder: Keller, Walter, Dipl.-Ing., D-8525 Marloffstein (DE); Peter, Fritz, Dipl.-Ing., D-8521 Spardorf (DE)

(56) Entgegenhaltungen:
- US-A- 3 095 252
- US-A- 4 181 850
- US-A- 4 201 430

## Beschreibung

Die Erfindung betrifft eine Datenübertragungsvorrichtung gemäß dem ersten Teil des Patentanspruches.

Eine Datenübertragungsvorrichtung dieser Art kann beispielsweise in einem Computertomographen zur Übertragung der Detektordaten Verwendung finden. Wird auch die Energie zum Röntgenstrahler, der zusammen mit dem Detektor auf einem Drehrahmen angeordnet ist, über Schleifringe übertragen, so ist eine Dauerrotation der Meßeinheit aus Röntgenstrahler und Detektor und damit eine sehr schnelle Abtastung mehrerer paralleler Schichten des Aufnahmeobjektes möglich.

Durch die US-A-4 201 430 ist ein Computertomograph bekannt, bei dem auf dem feststehenden Teil Einheiten zur Versorgung der Röntgenröhre auf dem rotierenden Teil angeordnet ist, wobei die Stromübertragung von diesen Einheiten zum rotierenden Teil über Schleifringe erfolgt, so daß eine Dauerrotation möglich ist. Die Stromversorgung des Detektor-Arrays erfolgt bei dem bekannten Computertomographen durch eine Stromversorgungseinheit auf dem rotierenden Teil. Hinsichtlich der Stromversorgung der weiter vorhandenen elektronischen Komponenten wie Verstärker, Multiplexer, ADC's usw. ist diesem Dokument im einzelnen nichts entnehmbar.

Der Erfindung liegt die Aufgabe zugrunde, eine Datenübertragungsvorrichtung gemäß dem ersten Teil des Patentanspruches so auszubilden, daß eine störungsfreie Datenübertragung sichergestellt ist, so daß die Datenübertragungsvorrichtung insbesondere in einem Computertomographen zur Übertragung der Detektordaten anwendbar ist.

Diese Aufgabe ist erfindungsgemäß gelöst durch die Merkmale des zweiten Teiles des Patentanspruches. Bei der erfindungsgemäßen Datenübertragungsvorrichtung werden alle elektronischen Komponenten auf dem rotierenden und auf dem feststehenden Teil von einer gemeinsamen Stromversorgungseinheit gespeist. Dadurch ist eine störungsfreie Datenübertragung gesichert.

Die Erfindung ist nachfolgend anhand eines in der Zeichnung dargestellten Ausführungsbeispieles näher erläutert. Es zeigen:
- Fig. 1: einen Computertomographen zur Erläuterung des Erfindungsgedankens, und
- Fig. 2: den für die Erfindung wesentlichen Teil des Computertomographen gemäß Fig. 1.

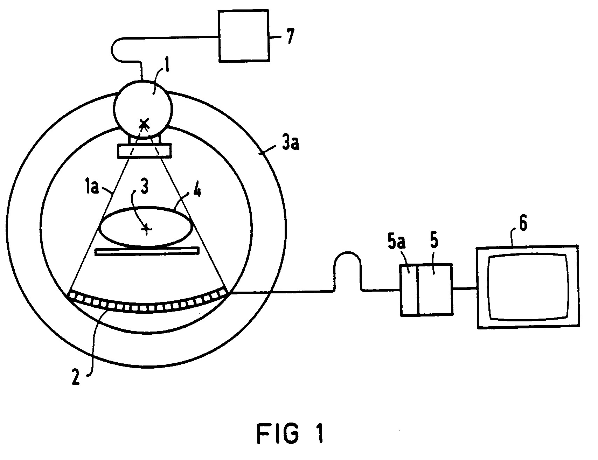

In der Fig. 1 ist ein Computertomograph mit einer Röntgenröhre 1 als Strahlenquelle und einem Strahlendetektor 2, der größenordnungsmäßig über 100, z.B. 512 einzelne Detektoren in einer Reihe angeordnet aufweist, dargestellt. Die Röntgenröhre 1 sendet ein fächerförmiges Strahlenbündel 1a aus, dessen Querschnittsausdehnung senkrecht zur untersuchten Schichtebene gleich der Schichtstärke und in der Schichtebene so groß ist, daß das ganze Aufnahmeobjekt von Strahlung durchsetzt wird. Der Strahlendetektor 2 ist um den Fokus der Röntgenröhre 1 gekrümmt. Die Meßanordnung 1, 2 ist um eine Achse 3, die etwa mit der Längsachse des Aufnahmeobjektes 4 zusammenfällt, mit Hilfe eines Drehrahmens 3a drehbar. Die Anzahl der Detektoren des Strahlendetektors 2 ist der gewünschten Bildauflösung entsprechend gewählt, so daß aufgrund einer Drehung der Meßanordnung 1, 2 von einem Computer 5 die Schwächungswerte einer Bildpunktmatrix der durchstrahlten Transversalschicht des Aufnahmeobjektes 4 berechnet und als Bild auf einem Sichtgerät 6 wiedergegeben werden können. Die Röntgenröhre 1 wird von einem Röntgengenerator 7 gespeist.

Jedem Detektorelement des Strahlendetektors 2 ist ein Meßkanal zugeordnet, der zum Computer 5 führt. In diesem Meßkanal sind Verstärkerschaltungen, Multiplexer und Analog-Digital-Wandler vorgesehen, die ein Datenerfassungssystem 5a bilden.

Eine schnelle Abtastung mehrerer paralleler Schichten des Aufnahmeobjektes 4 ist möglich, wenn der Drehrahmen 3a kontinuierlich rotieren kann, wobei die Liege mit dem Aufnahmeobjekt 4 durch den Drehrahmen 3a hindurch geschoben wird. Hierzu wird der Röntgenröhre 1 die Energie vom Röntgengenerator 7 über eine nicht dargestellte Schleifringanordnung zugeführt und auch die Daten der Detektorelemente des Strahlendetektors 2 werden über eine Schleifringanordnung zum Datenerfassungssystem 5a übertragen.

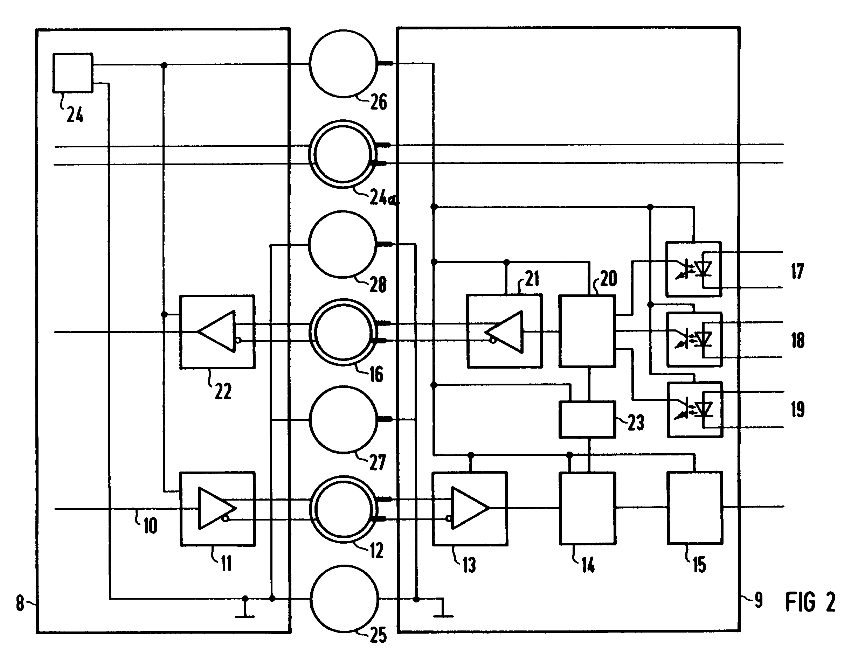

In der Fig. 2 ist der rotierende Teil schematisch mit 8 und der feststehende Teil mit 9 bezeichnet. Die Daten des Strahlendetektors 2 kommen auf einer Leitung 10 an und werden über einen Verstärker 11 und eine Schleifringanordnung 12 zu einem Verstärker 13 im feststehenden Teil 9 übertragen. Dem Verstärker 13 sind ein Regenerator 14 und ein Optokoppler 15 nachgeschaltet. Eine weitere Schleifringanordnung 16 dient zur Übertragung von Steuersignalen an den Eingängen 17, 18 und 19. Der Schleifringanordnung 16 sind ein Multiplexer 20 und ein Verstärker 21 vor- und ein Verstärker 22 nachgeschaltet. Ein Pulsgenerator 23 synchronisiert den Multiplexer 20 und den Regenerator 14. Zwischen den Eingängen 17 bis 19 und dem Multiplexer 20 liegen Optokoppler. Die Energie zum Röntgenstrahler 1 wird über eine Schleifringanordnung 24a übertragen.

Die Stromversorgung der Komponenten 11, 22 auf dem rotierenden Teil und 13, 14, 15, 20, 21, 23 auf dem feststehenden Teil 9 erfolgt durch eine gemeinsame Stromversorgungseinheit 24 auf dem rotierenden Teil 8. Zur Übertragung des Stromes von der Stromversorgungseinheit 24 zu den Komponenten 13, 14, 15, 20, 21, 23 auf dem feststehenden Teil 9 sind Schleifringanordnungen 25, 26, 27, 28 vorgesehen. Von der Stromversorgungseinheit 24 werden also alle Komponenten auf dem rotierenden Teil 8 und feststehenden Teil 9 gespeist, wodurch eine optimale, störungsfreie Datenübertragung sichergestellt ist.

## Patentansprüche

1. Datenübertragungsvorrichtung mit einer ersten Schleifringanordnung (12, 16) für die Datenübertragung sowie elektronischen Komponenten (11, 13, 14, 15, 20 bis 23) auf einem feststehenden Teil (9) und einem rotierenden Teil (8), **dadurch gekennzeichnet,** daß für alle Komponenten (11, 13, 14, 15, 20 bis 23) auf dem feststehenden Teil (9) und dem rotierendem Teil (8) eine gemeinsame Stromversorgungseinheit (24) auf dem rotierenden Teil (8) vorgesehen ist, die mit den Komponenten (13, 14, 15, 20, 21, 23) des feststehenden Teiles (9) über eine zweite Schleifringanordnung (25, 26, 27, 28) verbunden ist.

## Claims

1. Data transmission device having a first wiper ring arrangement (12, 16) for data transmission, and electrical components (11, 13, 14, 15, 20 to 23) on a fixed part (9) and a rotating part (8), characterised in that for all the components (11, 13, 14, 15, 20 to 23) on the fixed part (9) and the rotating part (8) there is provided a common current supply unit (24) on the rotating part (8) which is connected to the components (13, 14, 15, 20, 21, 23) of the fixed part (9) via a second wiper ring arrangement (25, 26, 27, 28).

## Revendications

1. Dispositif de transmission de données comportant un premier dispositif à bagues collectrices (12,16) pour la transmission de données ainsi que des composants électroniques (11,13,14,15,20 à 23) sur une partie fixe (9) et sur une partie tournante (8), caractérisé par le fait que pour tous les composants (11,13,14,15,20 à 23), situés sur la partie fixe (9) et sur la partie tournante (8), il est prévu, sur la partie tournante (8), une unité commune d'alimentation en courant (24), qui est connectée aux composants (13,14,15,20,21,23) de la partie fixe (9) par l'intermédiaire d'un second dispositif à bagues collectrices (25,26,27,28).
